(19) Europäisches Patentamt
European Patent Office
Office européen des brevets

(11) Veröffentlichungsnummer: **0 155 506**
**B1**

(12) # EUROPÄISCHE PATENTSCHRIFT

(45) Veröffentlichungstag der Patentschrift: **24.08.88**

(51) Int. Cl.[4]: **C 07 D 475/14**

(21) Anmeldenummer: **85101665.9**

(22) Anmeldetag: **15.02.85**

(54) **Verbessertes Verfahren zur Herstellung von Riboflavin.**

(30) Priorität: **22.02.84 DE 3406319**

(43) Veröffentlichungstag der Anmeldung: **25.09.85 Patentblatt 85/39**

(45) Bekanntmachung des Hinweises auf die Patenterteilung: **24.08.88 Patentblatt 88/34**

(84) Benannte Vertragsstaaten: **CH DE FR GB LI NL**

(56) Entgegenhaltungen: **US - A - 2 847 413**

(73) Patentinhaber: **BASF Aktiengesellschaft, Carl-Bosch-Strasse 38, D-6700 Ludwigshafen (DE)**

(72) Erfinder: **Grimmer, Johannes, Aeblerose Vey 11, DK-8500 Grenaa (DK)**
Erfinder: **Horn, Hans Christoph, Dr., Roemerstrasse 22, D-6715 Lambsheim (DE)**

**EP 0 155 506 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein verbessertes Verfahren zur Herstellung von Riboflavin (I; Vitamin $B_2$) durch Kondensation eines 4,5-Dimethyl-N-(D)-ribityl-2-phenylazo-anilins II mit Barbitursäure III in Gegenwart eines sauren Kondensationsmittels in einem organischen Lösungsmittel.

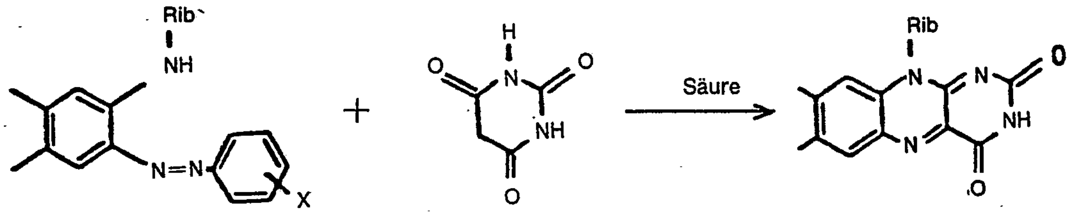


(II) (III) (I)

Rib = Ribityl X = H; p- oder o-Substituent wie -Cl, $-NO_2$ oder $-CH_3$.

Dieser letzte Schritt der Riboflavinsynthese ist – sieht man von der erfindungsgemäßen Verbesserung ab – aus mehreren Veröffentlichungen bekannt. Erstmals beschrieben wurde die Umsetzung von II mit III zu I in J. Am. Chem. Soc. 69 (1947), Seiten 1487 f. Als Reaktionsmedium wurde hierin insbesondere ein Gemisch aus Eisessig und Dioxan genannt. Da bei diesem Verfahren das relativ teure Lösungsmittel Dioxan in großen Mengen verwendet werden muß und zudem gegen Dioxan Bedenken wegen gesundheitsgefährdender Wirkung bestehen, ist dieses Verfahren für eine Riboflavinherstellung in technischem Maßstab nicht geeignet.

Eine ausführliche Untersuchung über die Eignung verschiedener organischer Säuren als Katalysator für die Umsetzung von II mit III wurde von Berezowski et al. im J. Gen. Chem. USSR 31 (1961), Seiten 3444 f. beschrieben. Als Lösungsmittel wurde siedendes Butanol verwendet. Die hierbei erzielten Ausbeuten von maximal etwa 70% befriedigen bei einem Verfahren im technischen Maßstab nicht, zumal, wenn es sich um den letzten Reaktionsschritt eines vielstufigen Verfahrens handelt, und da diese Ausbeuten auch nur bei Verwendung von größeren III-Überschüssen erzielt wurden.

Gemäß der CS-PS 127 303 wird II mit III in Gegenwart von Eisessig in Butanol oder Dioxan umgesetzt. Die erzielten Ausbeuten betragen maximal 78%.

Aus der JA-OS 7737/1963 ist die Umsetzung von II mit III in einem Gemisch aus Alkanolen mit Kp = 80 bis 120 °C und Eisessig an $Al_2O_3$ oder gereinigter Diatomeenerde bekannt. Die Ausbeuten betragen 67–69% der Theorie.

Nachteilig bei der Umsetzung von II mit III in Alkanolen ist, daß als Nebenreaktion eine teilweise Veresterung der Alkanole mit der Essigsäure stattfindet, wodurch einerseits zusätzlich zu dem reaktionsbedingten Kondensationswasser Wasser in das Reaktionsgemisch gelangt, welches die weitere Kondensation von II mit III stört und andererseits ein aufwendiges Regenerieren des Lösungsmittels notwendig ist. Außerdem verursachen die physikalischen Eigenschaften des Lösungsmittel-Katalysator-Systems sowie die Reaktions- und Kristallisationsbedingungen bei der Synthese eine geringere Reinheit des Riboflavins.

Aus der JA-OS 10151 ist weiterhin ein Verfahren zur Kondensation von II mit III in einem Gemisch aus einem Lösungsmittel wie Essigsäureethylester und Nitrobenzol oder einem nitrierten Alkan bekannt. Die Ausbeuten betragen 82–84%. Nachteilig an diesem Verfahren sind einerseits zu niedrigere Raum-Zeit-Ausbeuten sowie die Notwendigkeit der Mitverwendung von Nitrobenzol u.ä., welches einen sehr hohen Dampfdruck aufweist und äußerst giftig ist.

Aus der CS-PS 195 229 ist ein Verfahren zur Herstellung von I aus II und III in einem Gemisch aus Xylol, einem $C_3$-$C_5$-Alkanol, Eisessig und Essigsäureestern bekannt. Die Ausbeuten betragen 86–87,4% an einem relativ reinen I. Nachteilig an diesem Verfahren sind die aufwendige Aufarbeitung des eingesetzten Lösungsmittelgemisches, schlechte Raum-Zeit-Ausbeuten und Schwierigkeiten beim Abfiltrieren des Produktes, da I aus diesem Gemisch in relativ kleinen, schwer filtrierbaren Kristallen ausfällt.

Weiterhin ist in der JA-OS 3575/1957 ein Verfahren zur Herstellung von I durch Umsetzen von II mit III in einem Gemisch aus einem organischen Lösungsmittel und Eisessig an einem sauren Salz wie $SnCl_2$, $ZnCl_2$, $FeCl_2$, $AlCl_3$ oder $BiCl_3$ als Katalysator bekannt. Man erhält I-Ausbeuten über 80% der Theorie. Nachteilig an diesem Verfahren ist, daß die erwähnten guten Ausbeuten nur erzielt werden, wenn man mit einem Barbitursäureüberschuß von 75 bis 110% arbeitet, daß das erhaltene Riboflavin in jedem Fall einer mit Ausbeuteverlusten verbundenen Nachreinigung unterzogen werden muß und daß die Regeneration der Lösungsmittel und der Metallsalze recht zeit- und kostenaufwendig ist.

Es war daher die Aufgabe der Erfindung, ein Lösunsmittel zu finden, in welchem die säurekatalysierte Kondensation von II und III in guten Ausbeuten sowie in guten Raum-Zeit-Ausbeuten verläuft, aus dem I außerdem in möglichst hoher Reinheit auskristallisiert und das zusätzlich möglichst oft wiederverwendet und/oder auf möglichst einfache Weise regeneriert werden kann.

Es wurde nun überraschenderweise gefunden, daß bei Verwendung bestimmter aliphatischer Diol- oder Triolderivate als Lösungsmittel die Nachteile des Standes der Technik weitgehend beseitigt sind

und daß I in sehr guten Ausbeuten und sehr reiner kritalliner Form erhalten wird.

Gegenstand der Erfindung ist daher ein verbessertes Verfahren zur Herstellung von Riboflavin der Formel I

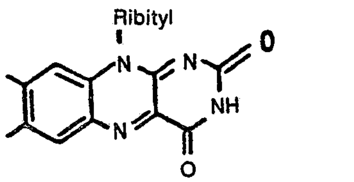

(I)

durch Kondensation eines 4,5-Dimethyl-N-(D)-ribityl-2-phenylazo-anilins der Formel II,

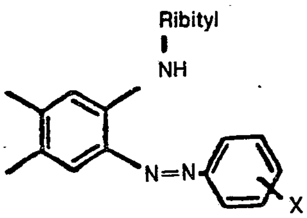

(II)

in der X für
-H, -Cl, $-NO_2$ oder $-CH_3$ in o- oder p-Stellung steht, mit Barbitursäure der Formel III

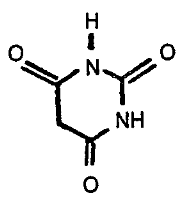

(III)

in Gegenwart eines sauren Kondensationsmittels in einem organischen Lösungsmittel, das dadurch gekennzeichnet ist, daß man die Umsetzung in einem aliphatischen Diol- oder TrioldERIVAT der allgemeinen Formel IV,

$$\begin{array}{l} R^1\text{-}CH\text{-}O\text{-}R^3 \\ \quad | \\ (CH_2)_n \\ \quad | \\ CH\text{-}O\text{-}R^4 \\ \quad | \\ R^2 \end{array} \qquad (IV)$$

in der
$R^1$ für -H oder $CH_3$- steht,
$R^2$ für -H oder $CH_3$- steht, oder wenn n für 0 steht, $CH_3$-CO-O-$CH_2$- bedeutet,
$R^3$ für $CH_3$-, $C_2H_5$- oder $CH_3$-CO- steht,
$R^4$ für $CH_3$-CO- steht oder für -H steht, wenn $R^3$ $CH_3$- oder $C_2H_5$- bedeutet, und
n für 0 oder 2, vorzugsweise 0, steht,
mit einem Siedepunkt von 80–180 °C oder einem Gemisch derselben als Lösungsmittel durchführt.

Mit besonderem Vorteil gestaltet sich das Verfahren, wenn man die Umsetzung in Ethylenglykoldiacetat oder in 1-Methoxy-2-propanol oder in einem Gemisch aus diesen Lösungsmitteln durchführt.

Bei Verwendung der erfindungsgemäßen Lösungsmittel werden nahezu quantitative Ausbeuten an I erzielt. Die Reinheit von I liegt immer bei mindestens 94%, meist aber über 95%.

Es war überraschend, daß auch hohe Ausbeuten und Reinheiten bei der Bildung von Riboflavin erzielt werden, wenn man ein Alkoxyalkanol, wie das 1-Methoxy-2-propanol, als Lösungsmittel für die Umsetzung verwendet. Bei diesem Reaktionsverlauf zeigt sich, daß ein Gemisch aus beispielsweise Methoxypropanol und Eisessig, ®Versaticsäure, Propionsäure oder Pivalinsäure kaum zur Veresterung neigt, so daß nur geringe Verluste an Lösungsmittel und Säure entstehen und damit ein kostspieliger Regenerierungsaufwand entfällt.

Völlig unerwartet ist eine Reinheitsverbesserung auf über 96% bei Anwendung einer Mischung aus den erfindungsgemäßen Acetaten, Alkoxyalkanolacetaten oder Alkoxyalkanolen in Gegenwart von Säurekatalysatoren.

Die Ausgangsverbindung II sowie deren Herstellung sind bekannt. Im allgemeinen wird man die billigste Verbindung dieser Reihe, nämlich das Phenylazoderivat einsetzen. Jedoch eignen sich prinzipiell auch Verbindungen, in denen die ortho- oder insbesondere die para-Stellung der Azophenylgruppe durch Substituenten wie Methyl, Chlor oder die Nitrogruppe substituiert sind. Die Ausgangsverbindungen müssen nicht speziell gereinigt werden, sondern können auch als Rohprodukte eingesetzt werden. Die Ausbeuteangaben beziehen sich dann auf im Ausgangsmaterial enthaltenes II.

Als inerte organische Verdünnungs- oder Lösungsmittel der allgemeinen Formel IV sind erfindungsgemäß insbesondere die Acetate mehrwertiger aliphatischer Alkohole, die Ethylenglykoldiacetat, Butan-1,4-diol-diacetat oder Glycerintriacetat sowie Alkoxyalkanolacetate, wie 2-Ethoxy-ethylacetat, und sogar Alkoxyalkanole, wie 1-Methoxy-2-propanol, geeignet. Mit ganz besonderem Vorteil verwendet man Ethylenglykoldiacetat oder 1-Methoxy-2-propanol oder ein Gemisch aus diesen beiden Lösungsmitteln. Die Menge des Lösungsmittels beträgt im allgemeinen etwa 2 bis 12 Liter pro Kilo II.

Als saure Kondensationsmittel kommen im Prinzip alle bisher in der Literatur für diese Umsetzung beschriebenen Säuren, vorzugsweise schwache organische Säuren wie Eisessig, Propionsäure, Phenylessigsäure, Benzoesäure, Trimethylessigsäure (Pivalinsäure), 2,2-Dimethyl-butansäure, 2,2-Dimethyl-pentansäure und 1-Methyl-cyclopentancarbonsäure in Betracht.

Technisch von besonderer Bedeutung sind der besonders billige Eisessig und im Handel erhältliche Gemische synthetischer Säuren, die im wesentlichen gesättigte tertiäre Carbonsäuren enthalten, wie vor allem die sogenannte Versatic®-10-Säure, eine synthetische $C_{10}$-Carbonsäure der Shell Chemie sowie ähnliche Produkte der Firma Esso, die unter dem Namen «Neosäuren» im Handel sind. Genannt seien hiervon beispielsweise die «Neopentansäure», die aus Hauptbestandteil Trimethylessigsäure enthält, und die «Neodecansäure», die in ihrer Zusammensetzung der Versatic®-10-Säure ähnlich sein dürfte.

Die Menge der Carbonsäure beträgt vorzugsweise 0,5 bis 6 Mol pro Mol II, was beispielsweise für Eisessig etwa 0,83 bis 1 kg pro kg II und für Versatic®-10-Säure 2,36 kg bis 2,85 kg pro kg II entspricht.

Es ist ein besonderer Vorteil des erfindungsgemäßen Verfahrens, daß man auch bei Einsatz äquimolarer Mengen von II und III deutlich höhere Ausbeuten als bisher erzielt. Diese Ausbeuten betragen etwa 85%, lassen sich aber noch auf weit über 90% erhöhen, wenn man III in einem bis zu 0,3-molaren Überschuß verwendet. Höhere Überschüsse stören nicht, erbringen jedoch keine merklichen Ausbeutesteigerungen mehr.

Die Reaktionstemperaturen liegen bei 80–120 °C. Vorzugsweise arbeitet man bei Temperaturen über 100 °C, also bei etwa 100 bis 115 °C sowie in einem entsprechend hochsiedenden Lösungsmittel. Bei 100 °C beträgt die Reaktionszeit etwa 5 bis 15 Stunden.

Mit Hilfe des erfindungsgemäßen Verfahrens erhält man Riboflavin aus II und III auf vorteilhafter Weise in sehr reiner Form sowie sehr guten Ausbeuten.

Beispiele 1 bis 9

Eine Mischung aus jeweils 40 g 4,5-Dimethyl-N-(D)-ribityl-2-phenylazoanilin (87%ig an den benötigten 2-Phenylazoisomeren; entsprechend 0,0966 mol), 16 g (0,125 mol) Barbitursäure und den aus der folgenden Tabelle ersichtlichen Mengen an Säuren und Lösungsmitteln wurde für die Dauer der angegebenen Reaktionszeit auf die angegebene Reaktionstemperatur erhitzt. Anschließend wurde das Reaktionsgemisch auf 60 °C abgekühlt und der ausgeschiedene Niederschlag zunächst abgesaugt, dann mit Methanol, 60 °C warmem Wasser und erneut mit Methanol gewaschen und bei 80–90 °C im Trockenschrank getrocknet. In der Tabelle sind die erzielten Ausbeuten und Reinheiten angegeben. Die prozentualen Ausbeuten beziehen sich dabei auf eingesetztes 100%iges 4,5-Dimethyl-N-(D)-ribityl-2-phenylazo-anilin und 100%iges Riboflavin.

Tabelle

| Beispiel | Lösungsmittel | [ml] | Säure [ml] | Rk-Zeit [h] | Temp. [°C] | Ausb. [g] | Reinh. [%] | Ausbeute [% d.Th.] |
|---|---|---|---|---|---|---|---|---|
| 1 | $CH_2$-O-Ac<br>$CH_2$-O-Ac | 200 | Eisessig<br>20 | 10 | 110–15 | 35,9 | 95,1 | 93,9 |
| 2 | $CH_2$-O-$C_2H_5$<br>$CH_2$-O-Ac | 180 | Eisessig<br>30 | 10 | 115 | 34,9 | 94,4 | 90,5 |
| 3 | $CH_2$-O-$CH_3$<br>CH-OH<br>$CH_3$ | 200 | Eisessig<br>20 | 10 | 110 | 32,8 | 96,1 | 86,6 |
| 4 | $CH_2$-O-$CH_3$<br>CH-OH<br>$CH_3$ | 120 | Versatic®-<br>10<br>säure<br>98 | 10 | 110–20 | 35,4 | 95 | 92,4 |
| 5 | $CH_2$-O-AC<br>CH-O-Ac<br>$CH_2$-O-Ac | 200 | Eisessig<br>20 | 3 | 120 | 32,9 | 94,9 | 85,9 |
| 6 | $CH_2$-O-Ac $CH_2$-O-$CH_3$<br>$CH_2$-O-AC + CH-OH<br>$CH_3$<br>100 ml | 100 | Eisessig<br>10 | 10 | 120 | 34,1 | 95,2 | 89,2 |
| 7 | $CH_2$-O-Ac $CH_2$-O-$CH_3$<br>$CH_2$-O-AC + CH-OH<br>$CH_3$<br>170 ml | 30 | Eisessig<br>10 | 10 | 120 | 35,8 | 96,8 | 95,3 |
| 8 | $CH_2$-O$CH_3$<br>CH-OH<br>$CH_3$ | 200 | Propion-<br>säure<br>20 | 10 | 110–15 | 32,7 | 96,3 | 86,6 |
| 9 | $CH_2$-O-Ac<br>$(CH_2)_2$<br>$CH_2$-O-AC | 200 | Eisessig<br>20 | 10 | 110–15 | 34,1 | 94,0 | 88,3 |

## Patentansprüche

1. Verfahren zur Herstellung von Riboflavin der Formel I

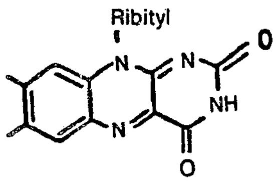

(I)

durch Kondensation eines 4,5-Dimethyl-N-(D)-ribityl-2-phenylazo-anilins der Formel II,

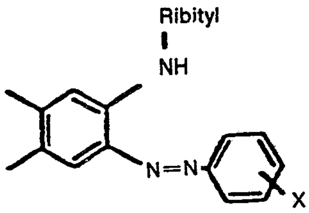

(II)

in der X für -H, -Cl, $-NO_2$ oder $-CH_3$ in o- oder p-Stellung steht, mit Barbitursäure der Formel III

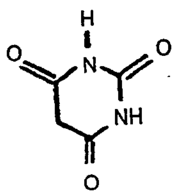

(III)

in Gegenwart eines sauren Kondensationsmittels in einem organischen Lösungsmittel, dadurch gekennzeichnet, daß man die Umsetzung in einem aliphatischen Diol- oder Triolderivat der allgemeinen Formel IV,

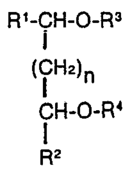

(IV)

in der
$R^1$ für -H oder $CH_3$- steht,
$R^2$ für -H oder $CH_3$- steht, oder wenn n für 0 steht, $CH_3$-CO-O-$CH_2$- bedeutet,
$R^3$ für $CH_3$-, $C_2H_5$- oder $CH_3$-CO- steht,
$R^4$ für $CH_3$-CO- steht oder für -H steht, wenn $R^3$ $CH_3$- oder $C_2H_5$- bedeutet, und
n für 0 oder 2 steht,

mit einem Siedepunkt von 80–180 °C oder einem Gemisch derselben als Lösungsmittel durchführt.

2. Verfahren gemäß Anspruch 1, dadurch gekennzeichnet, daß man die Umsetzung in einem aliphatischen Diol- oder Triolderivat der allgemeinen Formel IV

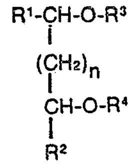

(IV)

in der n für 0 steht und die Reste $R^1$ bis $R^4$ die in Anspruch 1 angegebene Bedeutung haben, als Lösungsmittel durchgeführt.

3. Verfahren gemäß Anspruch 1, dadurch gekennzeichnet, daß man die Umsetzung in Ethylenglykoldiacetat oder einem Ethylenglykoldiacetat enthaltenden Gemisch als Lösungsmittel durchführt.

4. Verfahren gemäß Anspruch 1, dadurch gekennzeichnet, daß man die Umsetzung in 1-Methoxy-2-propanol oder einem 1-Methoxy-2-propanol enthaltenden Gemisch als Lösungsmittel durchführt.

## Claims

1. A process for the preparation of riboflavin of the formula I

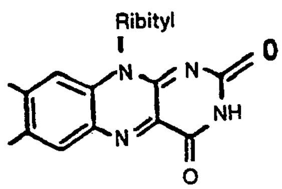

(I)

by condensation of a 4,5-dimethyl-N-(D)-ribityl-2-phenylazoaniline of the formula II

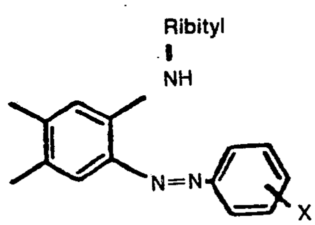

(II)

where X is -H, -Cl, $-NO_2$ or $-CH_3$ in the o- or p-position, with barbituric acid of the formula III

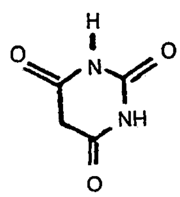

(III)

in the presence of an acidic condensing agent in an organic solvent, wherein the reaction is carried out in an aliphatic diol or triol derivative of the formula IV

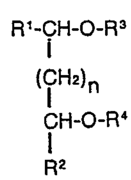

(IV)

where $R^1$ is -H or $CH_3$-, $R^2$ is -H or $CH_3$- or, when n is 0, is $CH_3$-CO-O-$CH_2$-, $R^3$ is $CH_3$-, $C_2H_5$- or $CH_3$-CO-, $R^4$ is $CH_3$-CO-, or is -H when $R^3$ is $CH_3$- or $C_2H_5$-, and n is 0 or 2, having a boiling point of 80–180 °C, or in a mixture of these as a solvent.

2. A process as claimed in claim 1, wherein the reaction is carried out in an aliphatic diol or triol derivative of the formula IV

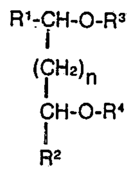

(IV)

where n is 0 and $R^1$ to $R^4$ have the meanings given in claim 1, as the solvent.

3. A process as claimed in claim 1, wherein the reaction is carried out in, as the solvent, ethylene glycol diacetate or a mixture containing this.

4. A process as claimed in claim 1, wherein the reaction is carried out in, as the solvent, 1-methoxy-propan-2-ol or a mixture containing this.

## Revendications

1. Procédé de préparation de riboflavine de formule

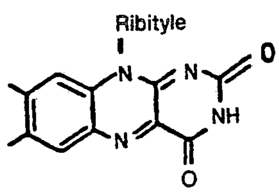

(I)

par condensation d'une 4,5-diméthyl-N-(D)-ribityl-2-phénylazoaniline de formule II

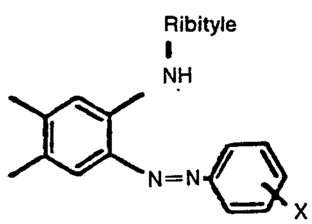

(II)

dans laquelle X représente -H, -Cl, -$NO_2$ ou -$CH_3$ en position ortho ou para, avec un acide barbiturique de formule III

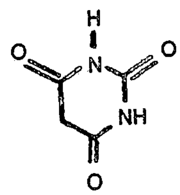

(III)

en présence d'un milieu de condensation acide dans un solvant organique caractérisé en ce que l'on mène la réaction dans un dérivé de diol ou de triol aliphatique de formule générale IV,

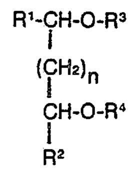

(IV)

dans laquelle

$R^1$ représente -H ou $CH_3$-,

$R^2$ représente -H ou $CH_3$-, ou quand n représente 0, $CH_3$-CO-O-$CH_2$-,

$R^3$ représente $CH_3$-, $C_2H_5$- ou $CH_3$-CO-,

$R^4$ représente $CH_3$-CO- ou -H, quand $R^3$ représente $CH_3$- ou $C_2H_5$-, et

n représente 0 ou 2,

ayant un point d'ébullition de 80 à 180 °C ou un de leurs mélanges, en tant que solvant.

2. Procédé selon la revendication 1, caractérisé en ce qu'on mène la réaction dans un dérivé de diol ou de triol aliphatique de formule générale IV

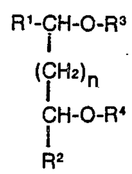

(IV)

dans laquelle n représente 0 et les restes $R^1$ à $R^4$ sont tels que définis dans la revendication 1, en tant que solvant.

3. Procédé selon la revendication 1, caractérisé en ce qu'on mène la réaction dans du diacétate d'éthylèneglycol ou dans un mélange contenant du diacétate d'éthylèneglycol en tant que solvant.

4. Procédé selon la revendication 1, caractérisé en ce qu'on mène la réaction dans du 1-méthoxy-2-propanol ou dans un mélange contenant de 1-méthoxy-2-propanol, en tant que solvant.